Europäisches Patentamt

European Patent Office

Office européen des brevets

(11) Veröffentlichungsnummer: **0 350 762**
**A1**

## (12) EUROPÄISCHE PATENTANMELDUNG

(21) Anmeldenummer: 89112146.9

(22) Anmeldetag: 03.07.89

(51) Int. Cl.4: **C07C 49/17 , C07C 45/00 , B01J 23/86**

(30) Priorität: 11.07.88 DE 3823458

(43) Veröffentlichungstag der Anmeldung:
17.01.90 Patentblatt 90/03

(84) Benannte Vertragsstaaten:
ES GR

(71) Anmelder: Henkel Kommanditgesellschaft auf Aktien
Postfach 1100 Henkelstrasse 67
D-4000 Düsseldorf 1(DE)

(72) Erfinder: Fleckenstein, Theo, Dr.
Pfitznerstrasse 9
D-4010 Hilden(DE)
Erfinder: Haberlandt, Klaus
Allensteinerstrasse 39
D-4100 Duisburg 46(DE)
Erfinder: Wahle, Bernd, Dr.
Am Heidt 24
D-4044 Kaarst 2(DE)
Erfinder: Göbel, Gerd, Dr.
Lindenstrasse 10
D-4006 Erkrath(DE)

(54) Verfahren zum Dehydrieren von aliphatischen 1,2-Diolen.

(57) Die Erfindung betrifft ein Verfahren zum Dehydrieren von aliphatischen 1,2-Diolen mit mindestens drei Kohlenstoffatomen zu 1,2-Ketonalkoholen in Gegenward von kupferhaltigen Katalysatoren bei Temperaturen zwischen 180 und 280 °C in der Gasphase. Um eine höhere Selektivität zu erreichen, wird erfindungsgemäß vorgeschlagen, daß die Diole 5 bis 60 Gew.-% Wasser, bezogen auf das Gesamtgewicht, enthalten und die Temperaturen zwischen 190 und 270 °C liegen.

EP 0 350 762 A1

## Verfahren zum Dehydrieren von aliphatischen 1,2-Diolen

Die Erfindung betrifft ein Verfahren zum Dehydrieren von aliphatischen 1,2-Diolen mit mindestens drei Kohlenstoffatomen zu 1,2-Ketonalkoholen in Gegenwart von kupferhaltigen Katalysatoren bei Temperaturen zwischen 180 und 280 °C in der Gasphase.

Verfahren zum Dehydrieren von aliphatischen 1,2-Diolen sind bekannt (DE-AS 23 13 957, DE-PS 850 608, DE-PS 756 063, FR-PS 977 009). In der DE-AS 23 13 957 wird beschrieben, daß Umsätze bis zu 86 % und Hydroxyaceton-Ausbeuten, bezogen auf umgesetztes Diol, bis zu 97 % (1. Tabelle) erreicht werden können. Bei technisch relevanten Langzeitversuchen werden jedoch tatsächlich nur Ausbeuten von 70 %, bzw. 94 %, bezogen auf umgesetztes Diol, erreicht. Ein weiterer Nachteil liegt bei den bekannten Verfahren darin, daß reine Einsatzstoffe verwendet werden, um lange Katalysatorstandzeiten zu erzielen. In den bekannten Verfahren wird ferner häufig ein Inertgas, z.B. Stickstoff oder Kohlendioxid zugegeben. Diese Maßnahme erschwert die Wasserstoff-Rückgewinnung.

Der Erfindung liegt die Aufgabe zugrunde, ein Verfahren der eingangs genannten Art zu entwickeln, das sich durch eine höhere Selektivität auszeichnet.

Diese Aufgabe wird erfindungsgemäß dadurch gelöst, daß die Diole 5 bis 60 Gew.-% Wasser, bezogen auf das Gesamtgewicht, enthalten und die Temperaturen zwischen 190 und 270 °C liegen.

Die Zugabe von Wasser bewirkt überraschenderweise eine Selektivitätssteigerung. Ein weiterer Vorteil gegenüber bisher bekannten Verfahren liegt in der Möglichkeit, wäßrige Ausgangsstoffe einzusetzen. Im nachhinein läßt sich das überraschende Ergebnis dadurch erklären, daß Wasser die Bildung dimerer Verbindungen zurückdrängt, ohne die Dehydrieraktivität des Katalysators zu beeinflussen. Da es sich beim Dehydrieren von 1,2-Diolen um einen endothermen Prozeß handelt, beeinflußt die Zugabe von Wasser das Verfahren auch dadurch positiv, daß die Abkühlung aufgrund der hohen Wärmekapazität von Wasserdampf verlangsamt wird.

Vorzugsweise wird bei dem erfindungsgemäßen Verfahren 1,2-Propandiol zu Hydroxyaceton dehydriert. 1,2-Propandiol ist ein Nebenprodukt der in der DE-OS 36 24 812 beschriebenen direkten Hydrierung von Triglyceriden. Dabei fällt wäßriges 1,2-Propandiol in Konzentrationen zwischen 35 Gew.-% und 62 Gew.-% an. Ein zusätzlicher Vorteil des erfindungsgemäßen Verfahrens liegt darin, daß das wäßrige 1,2-Propandiol ohne vollständigen Wasserabzug und damit energie- und rohstoffsparend zu Hydroxyaceton dehydriert werden kann. In der Verbindung mit dem Verfahren zur direkten Hydrierung von Triglyceriden gibt es den weiteren Vorteil, daß der beim Dehydrieren rückgewonnene Wasserstoff wieder zum Hydrieren weiterer Triglyceride verwendet werden kann.

Beim Dehydrieren von 1,2-Propandiol konnten bei einem 80 %igen Einsatzstoff und einem Kupferchromitkatalysator bei einer langen Betriebsdauer ein Umsatz von Hydroxyaceton zwischen 80 und 86 % erzielt werden.

Vorteilhaft ist, wenn ein wasserbeständiger Kupferchromitkatalysator verwendet wird. Diese Maßnahme erhöht die Standzeit des Katalysators.

Vorzugsweise enthalten die Diole 10 bis 40 Gew.-% Wasser, bezogen auf das Gesamtgewicht. Derartige Wasseranteile wirken besonders selektivitätssteigernd.

Vorteilhaft ist ferner, wenn die Temperaturen im Bereich zwischen 220 und 240 °C liegen.

Vorzugsweise wird bei dem erfindungsgemäßen Verfahren kein Inertgas zugegeben. Das hat zur Folge, daß der gebildete Wasserstoff nach einer Reinigung für Hydrierzwecke wiederverwendet werden kann. Dadurch wird das Verfahren wirdschaftlicher, insbesondere in Verbindung mit einem Hydriervorgang, dessen Reaktionsprodukt das Diol ist.

Als vorteilhaft hat sich ferner herausgestellt, wenn der Katalysator Kupferoxid auf einem Träger von Siliciumdioxid oder Zinkoxid enthält.

Das Verfahrensprodukt Hydroxyaceton wird in der Textilfärberei und in der Kunststoffindustrie verwendet. In der chemischen Industrie dient es als Zwischenprodukt zur Synthese von Heterozyklen und wertvollen Farbstoffen. In der Fotoindustrie wird Hydroxyaceton als Zusatz in Fixiermitteln eingesetzt. In der Kosmetik kann es als Ausgangsstoff für Bräunungsmittel und Geruchsvernichter verwendet werden.

Das erfindungsgemäße Verfahren, das durch die Zugabe von Wasser zu einer Umsatz- und Selektivitätssteigerung führt, wird im folgenden anhand von Ausführungsbeispielen näher beschrieben. Darin bedeuten die Abkürzungen PD Propandiol und HA Hydroxyaceton.

Beispiel 1

2

500 ml des in der DE-OS 36 24 812, Beispiel 2, beschriebenen Kupfer-Chromit-Katalysators in Form von Granulat der Größe 1-2 mm werden in einem Rohr von 2,5 cm Durchmesser fest angeordnet. Nach Reduktion mit 1 % $H_2$ in Stickstoff bei 150 -200 ° C wird von oben stündlich eine Mischung von 250 ml 1,2-Propandiol und 63 ml Wasser dampfförmig zudosiert. Die Reaktionstemperatur beträgt 240 ° C. Es wird lediglich ein geringer, zur Überwindung des Druckverlustes der Katalysatorschüttung benötigter Überdruck eingestellt.

Nach dem Abkühlen wird der gebildete Wasserstoff abgetrennt und das Produkt gaschromatographisch analysiert. Bei einem Umsatz von 79,9 % beträgt die Hydroxyaceton-Selektivität 97,6 %.

Beispiel 2

1 Liter des Kupfer-Chromit-Katalysators Cu-0203 T der Fa. Harshaw in Form von 1/8 Tabletten wird in einem Rohr von 3,5 cm Durchmesser wie in Beispiel 1 reduziert. Von oben wird stündlich eine Mischung von 500 ml 1,2 Propandiol und 125 ml Wasser dampfförmig zudosiert. Die Reaktionsbedingungen werden wie in Beispiel 1 eingestellt. Bei einem Umsatz von 81,9 % wird eine Hydroxyaceton-Selektivität von 98,3 % erreicht.

Beispiel 3

Als Vergleichsversuch wird das Verfahren wie in Beispiel 2, jedoch ohne Wasserzusatz, aber mit Zugabe von 2 m³ Stickstoff/h, durchgeführt. Der Umsatz beträgt 76,5 % und die Selektivität 97,4 %.

Beispiel 4

Der Einfluß der Wasserzugabe wird anhand der Tabelle deutlich. Bei folgenden Reaktionsbedingungen wird dehydriert.

| Reaktionstemperatur: | 200 ° C |
|---|---|
| LHSV: | 0,25 $h^{-1}$ |
| GHSV ($N_2$): | 1 000 $h^{-1}$ |
| P: | 4 bar |
| Katalysator: | siehe Beispiel 1 |

| 1,2-PD ml/h | $H_2O$-Zugabe ml/h | 1,2-PD-Umsatz % | HA-Selektivität % |
|---|---|---|---|
| 125 | - | 65,2 | 87,8 |
| 125 | 15 | 66,5 | 94,3 |
| 125 | 95 | 67,8 | 94,0 |

**Ansprüche**

1. Verfahren zum Dehydrieren von aliphatischen 1,2-Diolen mit mindestens drei Kohlenstoffatomen zu 1,2-Ketonalkoholen in Gegenwart von kupferhaltigen Katalysatoren bei Temperaturen zwischen 180 und 280 ° C in der Gasphase, dadurch gekennzeichnet, daß die Diole 5 bis 60 Gew.-% Wasser, bezogen auf das Gesamtgewicht, enthalten und die Temperaturen zwischen 190 und 270 ° C liegen.

2. Verfahren nach Anspruch 1, dadurch gekennzeichnet, daß 1,2-Propandiol zu Hydroxyaceton dehydriert wird.

3. Verfahren nach Anspruch 1, dadurch gekennzeichnet, daß ein wasserbeständiger Kupferchromit-Katalysator verwendet wird.

4. Verfahren nach einem der Ansprüche 1 bis 3, dadurch gekennzeichnet, daß die Diole 10 bis 40 Gew.-% Wasser, bezogen auf das Gesamtgewicht, enthalten.

5. Verfahren nach einem der Ansprüche 1 bis 4, dadurch gekennzeichnet, daß die Temperaturen im Bereich zwischen 220 und 240 °C liegen.

6. Verfahren nach einem der Ansprüche 1 bis 5, dadurch gekennzeichnet, daß kein Inertgas zugegeben wird.

7. Verfahren nach einem der Ansprüche 1 bis 6, dadurch gekennzeichnet, daß der Katalysator Kupferoxid auf einem Träger von Siliciumdioxid oder Zinkoxid enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-2 143 383 (R. W. MC NAMEE et al.) * Ansprüche 1-6 * | 1-5 | C 07 C 49/17 C 07 C 45/00 B 01 J 23/86 |
| Y | DE-C- 722 204 (IG FARBEN AG) * Beispiele 1,2 * | 1-5 | |
| A | EP-A-0 204 046 (INDUSTRIAL TECHNOLOGY RESEARCH INSTITUTE) * Ansprüche 1,10 * | 1 | |
| A | DE-B-2 347 097 (RUHRCHEMIE AG) * Anspruch 1 * | 1 | |
| A | US-A-1 955 882 (J. HILGER) * Beispiel, Anspruch 1 * | 1 | |
| A | DE-B-1 468 117 (JEFFERSON CHEMICAL CO. INC.) * Beispiel 1 * | 1 | |
| A,D | DE-B-2 313 957 (BASF AG) * Anspruch 1 * | 1 | RECHERCHIERTE SACHGEBIETE (Int. Cl.5) C 07 C 45/00 C 07 C 49/00 |

Der vorliegende Recherchenbericht wurde für alle Patentansprüche erstellt

| Recherchenort | Abschlußdatum der Recherche | Prüfer |
|---|---|---|
| BERLIN | 04-10-1989 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument

EPO FORM 1503 03.82 (P0403)